Europäisches Patentamt

European Patent Office

Office européen des brevets

(19)

(11) Veröffentlichungsnummer : **0 416 565 B1**

(12) **EUROPÄISCHE PATENTSCHRIFT**

(45) Veröffentlichungstag der Patentschrift :
**23.02.94 Patentblatt 94/08**

(51) Int. Cl.⁵ : **C08B 15/00,** B01D 71/10,
// A61M1:16

(21) Anmeldenummer : **90117027.4**

(22) Anmeldetag : **05.09.90**

(54) **Verfahren zur Herstellung von Desoxycelluloseverbindungen.**

(30) Priorität : **08.09.89 DE 3929883**

(43) Veröffentlichungstag der Anmeldung :
**13.03.91 Patentblatt 91/11**

(45) Bekanntmachung des Hinweises auf die
Patenterteilung :
**23.02.94 Patentblatt 94/08**

(84) Benannte Vertragsstaaten :
**AT BE CH DE DK ES FR GB GR IT LI LU NL SE**

(56) Entgegenhaltungen :
**EP-A- 0 300 250**

(56) Entgegenhaltungen :
**JOURNAL OF APPLIED POLYMER SCIENCE,
Band 33, Nr. 2, 5. Februar 1987, Seiten 247-282,
John Wiley & Sons, Inc., New York, US; R.K.
JAIN et al.:** "Thermal, morphological and
spectroscopic studies on cellulose modified
with phosphorus, nitrogen, sulphur and halogens"
**POLYMER, Band 28, Dezember 1987, Seiten
2317-2323, Butterworth & Co., Ltd, GB; C.L.
McCormick et al.:** "Derivatization of cellulose
in lithium chloride and N-N-dimethylacetamide solutions"

(73) Patentinhaber : **Akzo N.V.**
**Postbus 9300 Velperweg 76**
**NL-6800 SB Arnhem (NL)**

(72) Erfinder : **Diamantoglou, Michael Dr.**
**Kolpingstrasse 4**
**D-8765 Erlenbach (DE)**
Erfinder : **Kundinger, Ernst F., Dr.**
**Odenwaldring 28**
**D-6127 Breuberg-Neustadt (DE)**

(74) Vertreter : **Fett, Günter**
**Akzo Patente GmbH, Postfach 10 01 49**
**D-42097 Wuppertal (DE)**

EP 0 416 565 B1

## Beschreibung

Die Erfindung betrifft ein Verfahren zur Herstellung von Desoxycelluloseverbindungen aus Cellulose in aprotischen Lösungsmitteln mit LiCl und/oder $CaCl_2$.

Aus der Zeitschrift "Polymer" 1987, Vol. 28, December. Seiten 2317 - 2323 ist die Derivatisierung von Cellulose in Lithiumchlorid/N,N-Dimethylacetamid-Lösungen von McCormick und Callais beschrieben worden. Im Beispiel 16 wird die Herstellung von Chlordesoxy-Cellulose mit einem Substitutionsgrad von 2,3 beschrieben. Ein solches hochsubstituiertes Produkt läßt sich allenfalls im Gemisch mit reiner Cellulose zur Fäden oder Membranen verarbeiten.

In der EP-A-0 300 250 werden Cellulosederivate beschrieben, die dadurch hergestellt werden, daß Cellulose und/oder ein entsprechender wasserunlöslicher Celluloseäther in Dimethylacetamid und/oder N-methylpyrrolidon und/oder Wasser aktiviert und nach gegebenenfalls erfolgtem Austausch von Wasser durch Dimethylacetamid oder N-methylpyrrolidon nach Zugabe von Lithiumchlorid gelöst wird, das mit entsprechenden Anhydriden, Isocyanaten, Säurechloriden, Ketenen, Diketenen, Lactonen, Sulfonen in an sich bekannter Weise umgesetzt wird. Hierzu ist jedoch vorher eine Aktivierung der Cellulose bei relativ hohen Temperaturen, z.B. 150°C, erforderlich.

Im Journal of Applied Polymer Science, Band 33, Nr. 2 (1987), S. 247-282 ist ein Verfahren zur Herstellung von Cellulosederivaten beschrieben, bei dem beispielsweise Halogendeoxycellulosen durch Detosyloxylation von Cellulosetosylat hergestellt werden. Dies geschieht durch Erhitzen der entsprechenden Verdingungen in 1,4-Pentandion bei 125°C.

Aufgabe der vorliegenden Erfindung war es, Desoxycellulose-derivate herzustellen, die sich durch eine höhere Reaktions-fähigkeit der Substituenten als die von Chlordesoxycellulose auszeichnen.

Gelöst wird diese Aufgabe durch ein Verfahren, das dadurch gekennzeichnet ist, daß das Lösungsmittel neben LiCl oder $CaCl_2$ zusätzlich Bromide, Jodide, Rhodanide oder Thiocarbamate der Alkalimetalle enthält, wobei die Cellulose zunächst in dem Gemisch aus aprotischem Lösungsmittel und Salzen gelöst, bei Raumtemperatur eine Base und/oder ein basisches Salz zugegeben und dann ein Sulfonsäurehalogenid entsprechend dem gewünschten Substitutionsgrad zugesetzt und bei Temperaturen zwischen 16°C und 100°C zur Reaktion gebracht wird.

Durch das erfindungsgemäße Verfahren werden Desoxycelluloseverbindungen zugänglich, die außer einer Cl-Substitution noch eine Rest-Substitution mit der Sulfonsäure und eine teilweise Substitution mit Brom, Jod und/oder Rhodanid aufweisen. Das Verhältnis der verschiedenen Substituenten läßt sich zum einen durch das Verhältnis von LiCl und/oder $CaCl_2$ zu den Salzen mit anderem Anion, zum anderen durch die äußeren Reaktionsbedingungen einstellen. Durch die gemischte Substitution wird die Reaktionsfähigkeit der Desocycelluloseverbindung bei milden Reaktionsbedingungen deutlich erhöht.

Vorzugsweise wird als aprotisches Lösungsmittel N,N-Dimethylacetamid (DMAc) eingesetzt. Ebenso lassen sich N-Methylpyrrolidon, Ethylen- oder Propylenharnstoff oder Dimethylsulfoxid verwenden.

Bei Temperaturen unterhalb 15°C findet weitgehend nur eine Reaktion der Cellulose mit dem Sulfonsäurehalogenid zu Cellulosesulfonsäureester statt. Dieser reagiert oberhalb von 15°C mit den in der Lösung anwesenden Halogenid- oder Pseudohalogenid-Ionen zu gemischt substituierten Cellulosederivaten. Temperaturen oberhalb 100°C führen zu stark abgebauten, unbrauchbaren Cellulosederivaten.

Vorzugsweise wird deshalb das Sulfonsäurehalogenid bei Temperaturen zwischen 40°C und 70°C zur Reaktion gebracht.

Unter diesen Reaktionsbedingungen läßt sich die Reaktion sehr gut steuern und es lassen sich die gewünschten Substitutionsgrade bei gutem Umsatz und guter Selektivität herstellen. Vorzugsweise werden dazu 0,1 - 2 mol/100 g Cellulose an Sulfonsäurehalogenid zugesetzt.

Ein bevorzugtes Anwendungsgebiet der hergestellten Desoxycelluloseverbindungen ist die Herstellung von reaktionsfähigen cellulosischen Membranen.

Eine andere bevorzugte Anwendung der hergestellten Desoxycelluloseverbindungen ist die Immobilisierung von Enzymen, wobei diese an geformten Gebilden, wie Fäden, Folien, Membranen, aber auch an pulverförmigen Produkten erfolgen kann.

Ein wesentliches Einsatzgebiet für Membranen sind Dialysatoren, insbesondere für künstliche Nieren. Dabei ist die Biocompatibilität der Membranen von besonderem Interesse.

Neben dem Umstand, daß Dialysemembranen aus synthetischen bzw. natürlichen Polymeren bei ihrem Einsatz in künstlichen Nieren sehr leicht eine Gerinnung des Blutes hervorrufen können, die durch entsprechende medikamentöse Behandlung weitgehend verhindert wird, tritt bei der Behandlung eines Nierenkranken mit Dialysatoren, die Membranen aus regenerierter Cellulose enthalten, in der ersten Zeit der Dialysebehandlung ein vorübergehender Leukozytenabfall auf. Dieser Effekt wird als Leukopenie bezeichnet.

Leukopenie ist eine Erniedrigung der Leukozytenzahl (weiße Blutkörper) im Blutkreislauf. Die Zahl der weißen Blutkörper beim Menschen beträgt ca. 4000 bis 12000 Zellen/mm³.

Die Leukopenie bei der Dialyse ist am stärksten ausgeprägt 15 bis 20 Minuten nach Beginn der Behandlung, wobei die Neutrophilen (das sind die mit neutralen oder gleichzeitig mit sauren und basischen Farbstoffen anfärbbaren Leukozyten) fast vollständig verschwinden können. Danach erholt sich die Zahl der Leukozyten innerhalb etwa einer Stunde wieder auf fast den Ausgangswert oder übersteigt diesen.

Wird nach Erholung der Leukozyten ein neuer Dialysator angeschlossen, tritt wieder Leukopenie im gleichen Ausmaß ein.

Cellulose-Membranen verursachen eine ausgeprägte Leukopenie. Auch wenn die klinische Bedeutung der Leukopenie wissenschaftlich nicht geklärt ist, besteht doch der Wunsch nach einer Dialysemembran für die Hämodialyse, die den Effekt der Leukopenie nicht zeigt, ohne daß dadurch die anderen sehr erwünschten Eigenschaften von Dialysemembranen aus regenerierter Cellulose beeinträchtigt werden.

Bei der Hämodialyse mittels Membranen aus regenerierter Cellulose hat man neben der Leukopenie auch eine deutliche Komplement-Aktivierung festgestellt. Das Komplement-System innerhalb des Blutserums ist ein komplexes, aus vielen Komponenten bestehendes Plasmaenzym-System, das auf verschiedene Weise der Abwehr von Schädigungen durch eindringende fremde Zellen (Bakterien u.a.) dient. Wenn Antikörper gegen den eindringenden Organismus vorhanden sind, kann komplementspezifisch durch den Komplex der Antikörper mit antigenen Strukturen der Fremdzellen aktiviert werden, anderenfalls erfolgt auf einem Alternativ-Weg durch besondere Oberflächenmerkmale der Fremdzellen die Komplement-Aktivierung. Das Komplement-System beruht auf einer Vielzahl von Plasma-Proteinen. Nach Aktivierung reagieren diese Proteine spezifisch in einer bestimmten Reihenfolge miteinander und am Ende wird ein zellschädigender Komplex gebildet, der die Fremdzelle zerstört.

Aus einzelnen Komponenten werden Peptide freigesetzt, die Entzündungserscheinungen auslösen und gelegentlich auch unerwünschte pathologische Folgen für den Organismus haben können. Es wird angenommen, daß die Aktivierung bei Hämodialysemembranen aus regenerierter Cellulose über den alternativen Weg erfolgt. Objektiv festgestellt werden diese Komplement-Aktivierungen durch eine Bestimmung der Komplement-Fragmente $C_{3a}$ und $C_{5a}$.

In diesem Zusammenhang wird auf folgende Arbeiten hingewiesen: D.E. Chenoweth et al., Kidney International Vol. 24, Seite 764 ff, 1983, und D.E. Chenoweth, Asaio-Journal Vol. 7, Seite 44 ff, 1984.

Im Rahmen der vorliegenden Erfindung wurde die Komplement-Aktivierung anhand der Fragmente $C_{5a}$ beurteilt. Dazu wurden in vitro 300 ml heparinisiertes Blutplasma über einen Zeitraum von 4 Std. mit einem Plasmafluß von 100 ml/min durch einen Dialysator mit 1 m² effektiver Austauschfläche rezirkuliert. In dem Plasma wurden die $C_{5a}$-Fragmente mit Hilfe der RIA-Methode (Upjohn-Test) bestimmt. Die relative Komplement-Aktivierung für den jeweiligen Meßzeitpunkt wurde durch Bildung des Verhältnisses aus der Konzentration zum Zeitpunkt der Probenahme und dem Anfangswert in Prozent errechnet. Zur Bewertung wurde der Meßwert nach 4 Std. Rezirkulationszeit herangezogen. Flachmembranen werden mit heparinisiertem Blutplasma 3 Stunden inkubiert und anschließend die $C_{5a}$-Fragmente bestimmt.

Der Durchschnittspolymerisationsgrad DP wurde in einer Cuen-Lösung nach DIN 54270 bestimmt.

Die Erfindung wird anhand der nachfolgenden Beispiele näher erläutert:

**Beispiel 1**

In einem 1-l-Dreihalskolben wurden 48,6 g (0,30 Mol) Cellulose (DP = 650, gemessen im Lösungsmittel Cuen) in 697 g (8 Mol) Dimethylacetamid suspendiert und bei 145 °C 30 min lang unter Stickstoff aktiviert. Nach dem Abkühlen auf 100 °C wurden 64,8 g (1,53 Mol) LiCl und 64,8 g (0,75 Mol) LiBr zugesetzt, wobei die Temperatur um 5 - 10 °C anstieg; anschließend wurde rasch auf Raumtemperatur abgekühlt und über Nacht gerührt. Zur viskosen Lösung wurden nacheinander 60,6 g (0,60 Mol) Triethylamin und 85,7 g (0,45 Mol) Toluolsulfochlorid zugesetzt. Zur Vervollständigung der Reaktion wurde das Reaktionsgemisch 96 h bei 50 °C weitergerührt. Nach dem Erkalten wurde das Reaktionsprodukt mit Ethanol ausgefällt, mit Wasser und Ethanol gewaschen und im Vakuumtrockenschrank bei 50 °C getrocknet. Dabei wurden 59 g eines Produktes mit folgenden Spezifikationen erhalten:

| Cl-Gehalt: | 7,80 % | DS = 0,47 |
| Br-Gehalt: | 2,10 % | DS = 0,06 |
| S-Gehalt: | 3,70 % | |
| Resttosylierungsgrad: | | DS = 0,24 |

Aus diesem Derivat wurde eine DMAC/LiCl-Lösung mit 7 % Cellulosederivatgehalt hergestellt und zu Flachmembranen verarbeitet. Im Vergleich zu unmodifizierter Cellulose-Membranen ist die C5a-Aktivierung um 86 % vermindert.

**Beispiele 2-6**

Analog dem Beispiel 1 wurden durch Umsetzung der in DMAc/LiCl gelösten Cellulose mit Toluolsulfochlorid in Anwesenheit von Triethylamin und LiBr, LiJ oder LiSCN die in der Tabelle 1 aufgeführten Desoxycellulose-derivate synthetisiert.

## Tabelle 1:

## Desoxycellulosederivate

| Bei-spiel | Cl % | Cl DS | Br % | Br DS | J % | J DS | N % | SCN DS | Resttosy-lierungs-grad S % | Resttosy-lierungs-grad S DS |
|---|---|---|---|---|---|---|---|---|---|---|
| 2 | 4 | 0,22 | 1,2 | 0,03 | – | – | – | – | 2,8 | 0,17 |
| 3 | 3,1 | 0,16 | 5,5 | 0,13 | – | – | – | – | 1,1 | 0,06 |
| 4 | 3,2 | 0,16 | – | – | 2,5 | 0,04 | – | – | 1,5 | 0,08 |
| 5 | 3,8 | 0,20 | – | – | 4,6 | 0,07 | – | – | 1,3 | 0,08 |
| 6 | 2,3 | 0,12 | – | – | – | – | 2,5 | 0,34 | – | – |

**Beispiel 7**

A. Herstellung von Tosylcellulose

In einem 2-1-Dreihalskolben wurden 48,6 g Cellulose Buckeye V68 (0,3 Mol) in 835,8 g Dimethylacetamid suspendiert und 30 min lang unter Rückfluß gekocht. Nach dem Abkühlen des Gemisches auf 100°C wurden 87,6 g LiCl zugegeben. Unter weiterem Abkühlen auf Raumtemperatur entstand innerhalb einiger Stunden eine klare Celluloselösung. Anschließend wurden bei Raumtemperatur unter Rühren 73 g Triethylamin (0,72 Mol) sowie weitere 94,4 Dimethylacetamid und 3,7 g LiCl zugegeben und gelöst. 114,5 g p-Toluolsulfonsäurechlorid wurden in 188,9 g Dimethylacetamid gelöst und unter Kühlung des Reaktionsgemisches im Wasserbad während einer Stunde zugetropft. Das Reaktionsgemisch wurde noch 2,5 h gerührt und dann in kaltes Wasser gegossen. Das ausfallende Reaktionsprodukt wurde mehrmals mit Wasser und abschließend mit Ethanol gewaschen und getrocknet. Es wurden 87,4 g eines Produktes folgender Zusammensetzung erhalten:

```
C-Gehalt      48,4%
H-Gehalt       5,0%
Cl-Gehalt      0,95%      DS = 0,08
S-Gehalt      10,15%      DS = 1,0
```

B. Umsetzung der Tosylcellulose mit Natrium-dithio-diethylcarbamat

9,48 g Tosylcellulose mit DS = 1 (0,03 Mol) wurden in einem 0,25-1-Dreihalskolben in 47,2 g Dimethylacetamid gelöst. 5,13 g Natriumdithiodiethylcarbamat wurden in 37,8 g Dimethylacetamid gelöst und zur Lösung der Tosylcellulose gegeben. Das Reaktionsgemisch wurde 4 h bei 60°C gerührt und mehrere Stunden bei Raumtemperatur stehen gelassen. Anschließend wurde das Reaktionsprodukt durch Eingiessen in Wasser ausgefällt und mehrmals mit Wasser, Ethanol und abschließend mit Diethylether gewaschen. Nach dem Trocknen wurde, 68 g eines weißen, watteartigen Produkts folgender Zusammensetzung erhalten:
C-Gehalt    46,2%
H-Gehalt     5,9%
N-Gehalt     3,0%
S-Gehalt    19,9%
Nach Ausstreichen einer Lösung des Reaktionsproduktes in Dimethylacetamid und Verdunsten des Lösungsmittels wurde eine Flachmembran erhalten.

**Beispiel 8**

A. Herstellung von Chlordesoxycellulose

Analog zu Beispiel 7 wurde Tosylcellulose mit einem durchschnittlichen Substitutionsgrad DS = 1 hergestellt. In einem 2-1-Dreihalskolben wurden 75 g Tosylcellulose in 303 g Dimethylacetamid gelöst. Anschließend wurde eine Lösung von 32 g LiCl in 390 g Dimethylacetamid zur Lösung der Tosylcellulose gegeben. Das Reaktionsgemisch wurde auf 50°C erwärmt und bei dieser Temperatur 20 h gerührt. Das Reaktionsprodukt wurde durch Eingiessen der Lösung in Wasser ausgefällt und mehrmals mit Wasser und Ethanol gewaschen. Nach dem Trocknen wurden 52,8 g eines Produktes folgender Zusammensetzung erhalten.

```
C-Gehalt      43,7%
H-Gehalt       5,3%
CL-Gehalt     11,25%      DS = 0,61
S-Gehalt       4,0%       DS = 0,24
```

B. Umsetzung von Chlordesoxycellulose mit Natrium-dithio-diethylcarbamat

In einem 0,25-1-Dreihalskolben wurden 6,3 g (0,02 Mol) der oben genannten Chlordesoxycellulose in 75,6 g Dimethylacetamid gelöst. Nach Zugabe einer Lösung von 3,4 g Natrium-dithio-diethylcarbamat (0,02 Mol) in 28,3 g Dimethylacetamid wurde das Reaktionsgemisch auf 60°C erwärmt und 8 h lang bei dieser Temperatur gerührt. Das Reaktionsprodukt wurde durch Eingiessen der Lösung in Wasser ausgefällt und mehrmals mit Wasser und Ethanol gewaschen. Nach dem Trocknen wurden 6,6 g eines hellbeigen Pulvers folgender Zusammensetzung erhalten.
C-Gehalt    45%

H-Gehalt    6,2%
Cl-Gehalt   3,6%
N-Gehalt    2,7%
S-Gehalt    14,1%

Nach Ausstreichen einer Lösung des Reaktionsproduktes in Dimethylacetamid und Verdunsten des Lösungsmittels wurde eine Flachmembran erhalten.

**Beispiel 9 und 10**

Analog zu Beispiel 8 wurden Chlordesoxycellulose mit verschiedenen Natrium-dithio-carbamaten umgesetzt. Die Natriumsalze und die Zusammensetzung der Produkte sind aus Tabelle 2 ersichtlich.

## Tabelle 2:

## Umsetzungsprodukte aus Chlordesoxycellulose und Natriumdithiocarbamat

|  |  | Zusammensetzung der Reaktionsprodukte | | | | |
|---|---|---|---|---|---|---|
|  |  | % C | % H | % Cl | % N | % S |
| 9 | Natrium-dithio-morpholin-carbamat | 42,3 | 5,5 | 14,6 | 2,2 | 12,95 |
| 10 | Natrium-dithiohydroxy-ethylpiperazincarbamat | 43,3 | 5,5 | -- | 3,8 | 12,85 |

Die Reaktionsprodukte ließen sich aus Lösung zu Flachmembranen verarbeiten.

**Patentansprüche**

1.   Verfahren zur Herstellung von Desoxycelluloseverbindungen aus Celluloselösungen in aprotischen Lösungsmitteln, insbesondere Dimethylacetamid, die LiCl und/oder CaCl₂ enthalten, dadurch gekennzeichnet, daß das Lösungsmittel neben LiCl oder CaCl₂ zusätzlich Bromide, Jodide, Rhodanide oder Thiocarbamate der Alkalimetalle enthält, wobei die Cellulose zunächst in dem Gemisch aus aprotischem Lösungsmittel und Salzen gelöst, bei Raumtemperatur eine Base und/oder ein basisches Salz zugegeben und dann 0,1-2 mol/100g cellulose an einem Sulfonsäure-halogenid zugesetzt und bei Temperaturen zwischen 16°C und 100°C zur Reaktion gebracht wird.

2.   Verfahren nach Anspruch 1, dadurch gekennzeichnet, daß das Sulfonsäurehalogenid bei Temperaturen zwischen 40°C und 70°C zur Reaktion gebracht wird.

## Claims

1. Process for preparing deoxycellulose compounds from cellulose solutions in aprotic solvents, in particular dimethylacetamide, which contain LiCl and/or $CaCl_2$, characterized in that as well as LiCl or $CaCl_2$ the solvent additionally contains bromides, iodides, thiocyanates or thiocarbamates of alkali metals, first the cellulose being dissolved in the mixture of aprotic solvent and salts, a base and/or a basic salt being added at room temperature, and then 0.1-2 mol/100 g of cellulose being added of a sulphonyl halide and reacted at temperatures between 16°C and 100°C.

2. Process according to Claim 1, characterized in that the sulphonyl halide is reacted at temperatures between 40°C and 70°C.

## Revendications

1. Procédé de préparation de composés de type désoxycellulose, à partir de solutions de cellulose dans des solvants aprotiques, en particulier du diméthylacétamide, contenant du LiCl et/ou du $CaCl_2$, caractérisé en ce que le solvant contient en outre, en plus du LiCl et du $CaCl_2$, des bromures, des iodures, des thiocyanates ou des thiocarbamates de métaux alcalins, et dans lequel on dissout d'abord la cellulose dans le mélange de solvant aprotique et de sels, on y ajoute une base et/ou un sel basique, à la température ambiante, puis on y ajoute de 0,1 à 2 moles d'un halogénure d'acide sulfonique pour 100 g de cellulose et on le fait réagir à une température située entre 16°C et 100°C.

2. Procédé conforme à la revendication 1, caractérisé en ce que l'on fait réagir l'halogénure d'acide sulfonique à une température située entre 40°C et 70°C.

7